# EUROPEAN PATENT SPECIFICATION

(11) **EP 4 010 309 B1**
(45) Date of publication and mention of the grant of the patent: **18.11.2026**
(21) Application number: 20750877.1
(22) Date of filing: 06.07.2020
(51) Int. Cl.: C07C 45/50, C07C 47/02

(54) **HYDROFORMYLATION SYSTEM WITH VENT REACTOR RECYCLE TO PRIMARY REACTOR**
HYDROFORMYLIERUNGSSYSTEM MIT RÜCKFÜHRUNG DES ENTLÜFTUNGSREAKTORS ZUM PRIMÄRREAKTOR
SYSTÈME D'HYDROFORMYLATION DOTÉ D'UN RECYCLAGE D'UN RÉACTEUR D'ÉVENT VERS UN RÉACTEUR PRIMAIRE

(30) Priority: 09.08.2019 US 201962884727 P
(43) Date of publication of application: 15.06.2022
(73) Proprietor: Oxea Corporation, Houston, TX 77079 (US)
(72) Inventor: ZALMAN, Kyle, J., Needville, TX 77461 (US); HEUWES, Markus, Bay City, TX 77414 (US); BISCHOFF, Christopher, J., Bay City, TX 77414 (US)
(74) Representative: Michalski Hüttermann & Partner mbB
(86) International application number: PCT/US2020/040875
(87) International publication number: WO 2021/029983

(56) References cited:
- US-A- 5 367 106

## Description

### Claim for Priority

This application is based on United States Patent Application No. 16/920,856 filed July 06, 2020. United States Patent Application No. 16/920,856 is based on United States Provisional Application No. 62/884,727 filed August 09, 2019.

### Technical Field

The present invention relates to hydroformylation of olefins to make aldehydes utilizing a vent reactor with catalyst and product recycle to a primary reactor from a liquid vapor separator associated with the vent reactor.

### Background

Processes for hydroformylating an olefin to prepare a carbonyl derivate containing one carbon atom more than the starting olefin by reacting the olefin with carbon monoxide and hydrogen, which mixture is also known as synthesis gas (syngas), in the presence of a Group VIII metal, e.g., rhodium, in complex combination with an organic ligand and carbon monoxide are well known in the art (referred to as "OXO" processes) and have enormous industrial importance:

RHC=CH₂ + H₂ + CO → RH₂C-CH₂-CHO

wherein R is an organic radical. Commercial olefin feedstocks which may be utilized are ethylene, propylene, and 1- and 2-butenes, pentenes, hexenes and the like. In general, any suitable olefin having a C2 (ethylene) to C6 (hexene) carbon content may be used. A single methyl branch at the olefinic carbon of a terminal olefin reduces its reaction rate by a factor of ten, but the effect of a branch diminishes as its distance from the double bond increases. Some C6 feedstocks which exhibit different reactivities include: 1-hexene; 4-methyl-1-pentene; 2-hexene; 4-methyl-2-pentene; 2-methyl-1-pentene; 2-methyl-2-pentene; and 2,3-dimethyl-2-butene.

United States Patent No. 5,367,106 to Unruh et al. (1994) discloses an OXO process with a coupled vent reactor for making aldehydes. United States Patent No. 9,695,098 to Miller et al. discloses a hydroformylation system utilizing a vent reactor with product recycle from the vent reactor to a common product/catalyst separator. *See, also,* United States Patent No. 5,105,018 to Miyazawa et al.*,* United States Patent No. 9,067,876 to Becker et al.*,* United States Patent No. 4,593,127 to Bunning et al.*,* as well as United States Patent No. 9,688,598 to Eisenschmid et al.

Because substantial amounts of the unreacted components are in the various streams, it is important to operate the systems in ways that maximize product recovery and minimize waste of raw material. Capital and energy operating costs are likewise important to efficient operation and there is an ongoing need in the art for improvements which reduce capital and operating costs.

### Summary of Invention

The present invention is directed to an improvement in a hydroformylation system for making aldehydes from olefins and syngas having a primary reactor and a vent reactor receiving unreacted olefin and syngas from the primary reactor and generating additional product aldehydes. The improvement includes a liquid vapor separator receiving the output from the vent reactor and returning a liquid stream comprising catalyst and additional product aldehyde from the vent reactor to the primary reactor.

Details are provided in the description following and in the appended **Figures.**

It will be appreciated from the discussion which follows that advantages of the invention include lower capital and energy operating costs since a flasher, flasher pre-heater and a receiver vessel suitable for the flasher can be eliminated. Moreover, olefin gas loss points through vents are reduced, reducing raw material costs and reducing waste emissions. With the inventive system the catalyst life is extended since the catalyst remains under pressure with syngas and reactant olefin, reducing deactivation and precipitation of the catalyst complex.

### Brief Description of Drawings

The present invention is described in connection with the **Figures** in which:
**Figure 1** **is** a schematic diagram illustrating an embodiment of the system of the invention with liquid recycle from a vent reactor to a primary reactor through a liquid receiver;
**Figure 2A** is a schematic view in elevation and section of an integrated vent reactor/liquid vapor separator incorporated into the system of **Figure 1****;** and
**Figure 2B** is a schematic plan view in section of the integrated vent reactor/liquid vapor separator of **Figure 2A****.**

### Detailed Description

It is to be understood that the particular hydroformylation reaction conditions employed in either the primary reactor or the vent reactor or the secondary reaction process are not critical to the subject invention and may be varied widely and tailored to meet the individual needs of the particular reactors utilized as well as to produce the particular aldehyde product desired.

Group VIII catalyst metals include rhodium, ruthenium, palladium, osmium, iridium, and combinations thereof, particularly, rhodium and ruthenium and especially rhodium, are employed in organometallic complexes as catalysts for the reaction of syngas with olefins to form aldehyde derivatives of the olefins which have one more carbon atom than the parent olefin. A wide range of olefinic feedstocks can be employed, including substituted olefins. While the present invention is broadly applicable to the hydroformylation of olefins of 2-20 carbon atoms, its most useful application are especially with alpha-olefins of 2 to 8 carbon atoms. The system and process is particularly suitable for hydroformylating ethylene and propylene to propionaldehyde and n-butyraldehyde respectively. In this invention, the same catalyst complex and olefin are used for both the primary reactor and vent reactor. The catalyst is circulated continuously between the two reactors and the unconverted olefin in the primary reaction process is introduced into the vent reactor which may be a plug-flow or back mixed reactor for conversion of the unreacted olefin to aldehyde.

The hydroformylation processes are typically carried out at superatmospheric pressure, typically under a partial pressure of about 4 to 20 atmospheres of hydrogen and carbon monoxide combined and with the molar ratio of hydrogen to carbon monoxide being in the range of about 0.5:1 to 10:1.

The hydroformylation reaction temperature in the reactors is usually within range of 80°C to 170°C, preferably in the range from about 120°C to about 140°C.

The liquid reaction medium or catalyst solution which is employed comprises, (a) the catalyst complex, (b) typically, an excess of the organic ligand employed in forming the complex over and above the mount required to complex the metallic component of the catalyst, (c) the hydroformylation reaction product along with by-products typically resulting from undesired condensation of the hydroformylation product aldehyde with itself, (d) a quantity of the olefin being hydroformylated, in an amount varying with the molecular weight of said olefin (the proportion of liquid olefin in the reaction medium usually being greater with high molecular weight olefins than with lower alkanes such as ethylene), and (e) optionally an inert reaction solvent.

The catalytic metal is complexed with hydrogen and carbon monoxide as well as with an organic ligand. While many organic ligands can be employed, those of particular significance comprise either monodentate or polydentate triorganophosphines, triorganophophites, triorganoarsines, or triorganostibines, with the phosphines and phosphites being of particular industrial importance. Simple monodentate phosphines and phosphites, as exemplified by triphenylphosphine and triphenylphosphite, are commonly employed industrially. However, polydentate ligands have advantages in that the large excesses of ligand which are often used with the monodentate ligands are not needed. The catalytic complex can be formed in situ in the hydroformylation reactor, or it can be preformed.

The concentration of catalyst to be maintained in the hydroformylation reaction medium is not critical to the successful employment of the present invention. Typically, however, when the catalytic metal is rhodium and when the ligand is triphenylphosphine, the liquid reaction medium will contain about 0.01 to 1.0% rhodium and up to about 20% or more triphenylphosphine by weight where suppression of iso-aldehydes is desired. **In** hydroformylating ethylene, the iso-aldehydes problem does not exist, and low ligand concentrations can be employed. **In** the absence of the added inert reaction solvent, the triphenylphosphine content in hydroformylating propylene, for example, may be as high as about 40%.

Inert solvent, which is optionally used in the reaction system, is not overly critical so long as it be miscible with the catalyst system and with the reactants and reaction products, low in volatility so as to facilitate stripping reaction product and by-products from it, and, of course, either chemically inert in the hydroformylation reaction system or else forming in that system a derivative which is, itself, inert while still fulfilling the other named requirements. (That is, a suitable solvent could be one which might undergo hydrogenation in the reactor and then in the hydrogenated form, be inert to further reaction.) Molecular weight is not a significant factor in the reaction solvents except as it relates to volatility, relatively high molecular weight being desired, of course, to facilitate retention of the inert solvent as a heavy end while the reaction products are stripped. It is already known to employ any of a large number of inert liquids including, for example, alkyl-substituted benzenes; pyridine and alkyl-substituted pyridines; tertiary amines; high-boiling esters such as dialkyldicarboxylates and triorganophosphates, as well as esters of polyols such as trimethylolpropane and pentaerythritol; ketones; alcohols such as the butanols; nitriles such as acetonitriles; and hydrocarbons including both saturated hydrocarbons such as kerosene, mineral oil, cyclohexane, naphtha, etc. and aromatics such as biphenyl. It is taught in United States. Patent No. 4,151,209 that particularly useful solvents include triphenylphosphine oxide and polyglycols, e.g., polyethylene glycol and polypropylene glycol, which have molecular weights of at least about 500.

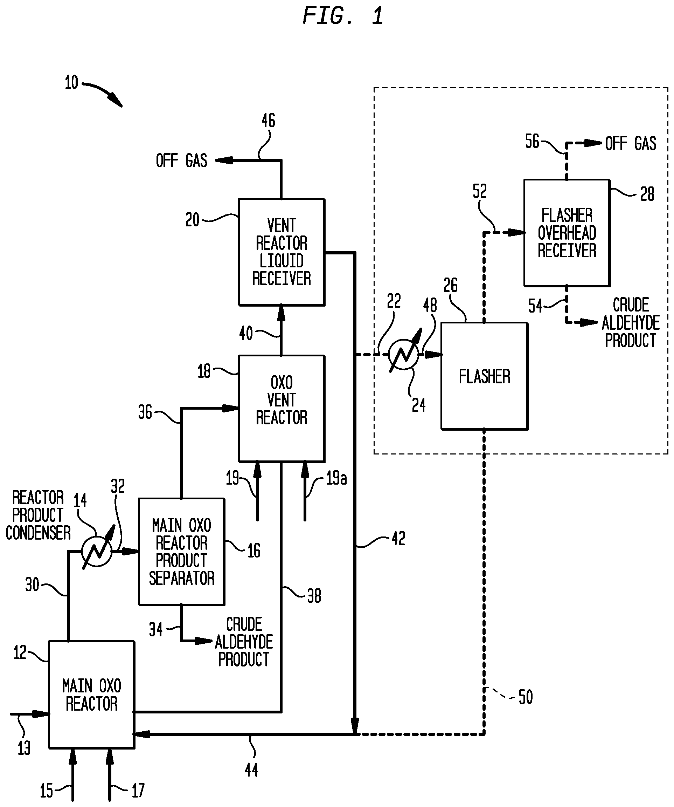

Referring to **Figure 1****,** there is shown a hydroformylation system 10, including a primary OXO reactor **12,** a condenser **14,** a main OXO reactor product separator **16,** a vent reactor **18** and a vent reactor liquid receiver **20.**

Also shown in **Figure 1** are components in a conventional system surrounded by a dashed line border which can be eliminated by way of the present invention. Conventional components include a feed line **22,** a flasher pre-heater **24,** a flasher **26,** as well as a flasher overhead receiver **28.**

In operation, flow of the various streams is in the direction indicated by the arrows in **Figure** 1. Primary OXO reactor **12** is fed with catalyst, syngas and olefin reactant at **13, 15** and **17** which are reacted to provide an aldehyde containing vaporous product stream which exits reactor **12** through line **30** and is provided to a condenser **14** where the product is cooled and provided to separator **16** through line **32.** In separator **16,** the liquid is withdrawn from the system as crude product at **34** and a vapor vent stream is provided to vent reactor **18** through line **36.**

Optionally, reactor **18** is provided with additional syngas and reactant olefin, as may be required for optimal operation, as indicated by arrows **19, 19a.**

Vent reactor **18** is coupled to reactor **12** via line **38** which provides a catalyst from the primary reactor to vent reactor **18.** That is, a portion of the liquid catalyst solution containing the rhodium-complex catalyst, any free organophosphorus ligand, solvent, a small amount of product aldehyde and any liquid heavy end product is continuously withdrawn from the primary OXO reactor. The withdrawn portion of the liquid catalyst solution is directed to the vent reactor which may be a plug-flow or back mixed reactor where it is mixed with the non-condensed vent gas separated from the product taken overhead from separator **16.** Additional syngas is typically added to the vent gas prior to mixing with the liquid catalyst solution in the vent reactor.

In vent reactor **18,** reactant olefin from main OXO separator **16** is converted to additional aldehyde product. The output from reactor **18,** preferably the entire output, including vapor, dissolved gasses, solvent and heavy ends circulating in the system are provided to receiver **20** via line **40** with or without additional cooling. The liquid from receiver **20,** including catalyst, crude aldehyde product, dissolved gasses, solvent and heavy ends circulating in the system, is recycled to primary reactor **12** by way of lines **42, 44.** Offgas is vented at **46.** The product aldehyde from the vent reactor may thus be recovered from the main separator **16.**

The system of the invention differs from conventional operation of a hydroformylation system which typically contains the items shown in dashed lines and/or within the dashed lines shown in **Figure 1****.** In particular, conventional practice involves providing liquid output from receiver **20** to a flasher preheater **24** through line **22** (line **42** not being present in a conventional system). Preheater **24** feeds flasher **26** through line **48,** where the product aldehyde is flashed off and the remaining liquid, including catalyst and any solvents, and heavies circulating in the system are returned to reactor **12** via lines **44, 50.** Flashed vapor from flasher **26** is provided to flasher overhead receiver **28** via line **52** where crude aldehyde product is drawn off through line **54** and vent gasses removed at **56.**

In the embodiment illustrated in **Figure 1****,** the product separators or receivers may be simply knock-out drums with or without cooling preceding the separator or receiver. Cooling may be provided by way of a separate condenser, such as condenser **14** or may be integral with the separator or receiver.

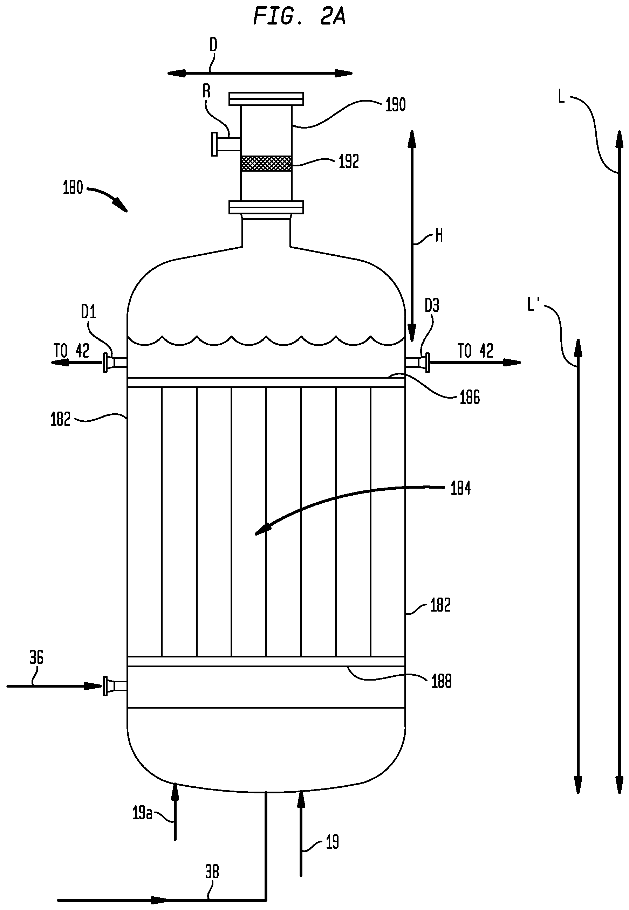

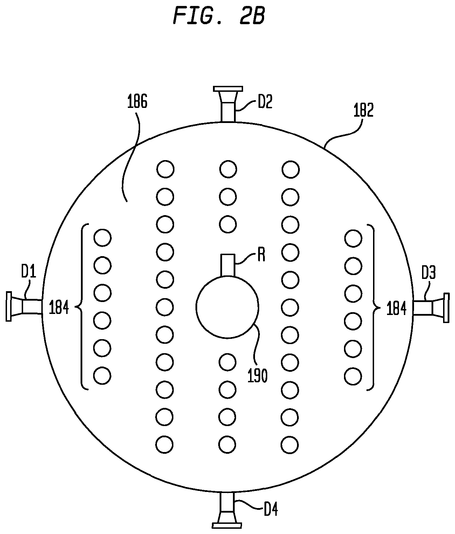

One preferred embodiment of the invention includes an integrated vent reactor/ liquid vapor separator as is shown in **Figures 2A** and **2B** which is incorporated into system **10** of **Figure 1****.** In **Figure 2A** there is shown schematically in elevation and section an integrated vent reactor/liquid vapor separator **180** used in the system of **Figure 1** instead of vent reactor **18** and separate liquid receiver **20.** Vent reactor/liquid vapor separator **180** includes a shell **182** with a plurality of tubes indicated at **184,** an upper tube sheet **186,** a lower tube sheet **188,** a plurality of liquid exit nozzles, **D1, D2, D3** and **D4.** Also provided is a vent gas exit manifold **190** with a vent gas exit nozzle **R,** and a mist eliminator **192.** Vent reactor/liquid vapor separator **180** is essentially a tube in shell heat exchanger type reactor (tube side reactor) with a vapor disengaging space at the top.

**Figure 2B** is a plan view of vent reactor/liquid vapor separator, in section, just above upper tube sheet **186** showing liquid exit nozzles **D1, D2, D3** and **D4** which are equally spaced around the periphery of shell **182.**

Vent reactor/liquid vapor separator **180** has a length to diameter ratio **(L/D)** in the range of from about 2 to 4, preferably around 3 and defines a liquid disengaging height, **H,** above a liquid level in the reactor such that the ratio of **H/L** is from about 0.1 to about 0.4, preferably around 0.2.

Vent reactor/liquid vapor separator **180** is used in system **10** of **Figure 1****,** coupled to the main OXO reactor **12** by way of line **38** to receive the **liquid** catalyst solution and coupled to separator **16** via line **36** to receive the vent stream. Optionally, vent reactor/liquid vapor separator **180** receives additional syngas and reactant olefin as indicate by arrows **19, 19a.**

When operating vent reactor/liquid vapor separator **180** reacts the unreacted vent gas received via line **36** in tubes **184** of the reactor, while maintaining a liquid level **L'** which is slightly above nozzles **D1, D2, D3** and **D4.** Vent gas is withdrawn via nozzle R after passing through mist eliminator **192.** Vent reactor/liquid vapor separator **180** is operated under substantially the same conditions as reactor **12;** that is, at temperatures of from 80°C to 170°C, preferably from 120°C to 140°C and at combined partial pressures of carbon monoxide and hydrogen of from 4 atmospheres to 20 atmospheres such that recycling to the main reactor is facilitated. When we refer to "substantially the same temperature" and/or "substantially the same pressure", such reference includes within 10% of the average value of the parameters within the recited apparatus components or as part of the recited process steps where pressure is on an absolute basis and temperature is on a Celsius scale.

Liquid is withdrawn from vent reactor/liquid vapor separator **180** via nozzles **D1-D4,** including catalyst, crude aldehyde product, dissolved gasses, solvent and heavy ends circulating in the system is recycled to primary reactor **12** by way of lines **42, 44.** The product aldehyde from the vent reactor/liquid vapor separator **180** may thus be recovered from main separator **16.**

The invention is defined in the following claims.

It is appreciated from the foregoing that advantages of the invention include lower capital costs since the flasher, flasher pre-heater and a receiver vessel can be eliminated. Moreover, olefin gas loss points through vents are reduced, reducing operating costs. Moreover, with the inventive system the catalyst life is extended since the catalyst remains under pressure with syngas and reactant olefin, reducing deactivation and precipitation of the catalyst complex.

In view of the foregoing discussion, relevant knowledge in the art and references discussed above in connection with the Background of the Invention and detailed description further description is deemed unnecessary. In addition, it should be understood from the foregoing discussion that aspects of the invention and portions of various embodiments may be combined or interchanged either in whole or in part. Furthermore, those of ordinary skill in the art will appreciate that the foregoing description is by way of example only, and is not intended to limit the invention.

## Claims

1. A hydroformylation system for making aldehydes comprising:
(a) a primary reactor provided with catalyst feed, syngas feed and olefin feed adapted to convert the olefin and syngas to product aldehyde;
(b) a first liquid vapor separator coupled to the primary reactor for receiving output therefrom, adapted to separate the product aldehyde into a crude aldehyde product stream and a vent stream containing syngas and unreacted olefin;
(c) a vent reactor coupled to the first liquid vapor separator to receive the vent stream therefrom, the vent reactor also being coupled to the primary reactor which is configured to provide catalyst thereto, wherein the vent reactor is operative to convert unreacted olefin in the vent stream from the first liquid vapor separator to additional product aldehyde;
(d) a second liquid vapor separator coupled to the vent reactor so as to receive the entire output from the vent reactor and adapted to separate the output from the vent reactor into a liquid recycle stream containing additional product aldehyde and catalyst as well as another vent stream, the second liquid vapor separator also being coupled to the primary reactor so as to provide the recycle stream thereto;
whereby catalyst from the second liquid vapor separator is recycled to the primary reactor and additional product aldehyde from the second liquid vapor separator is provided to the primary reactor and is recovered in the crude aldehyde product stream from the first liquid vapor separator.

2. The hydroformylation system for making aldehydes according to Claim 1, wherein the hydroformylation system consists of: (a) the primary reactor; (b) the first liquid vapor separator; (c) the vent reactor; and (d) the second liquid vapor separator.

3. The hydroformylation system for making aldehydes according to Claim 1, wherein the system has a single crude aldehyde product outlet.

4. The hydroformylation system for making aldehydes according to Claim 1, wherein the vent stream from the first liquid vapor separator comprises product aldehyde, syngas, and unreacted olefin.

5. The hydroformylation system for making aldehydes according to Claim 1, wherein the vent reactor is a plug flow reactor.

6. The hydroformylation system for making aldehydes according to Claim 1, wherein the vent reactor is a back mixed reactor.

7. The hydroformylation system for making aldehydes according to Claim 1, wherein the second vapor liquid separator is integrated with the vent reactor within a common shell.

8. The hydroformylation system for making aldehydes according to Claim 7, wherein the primary reactor, the vent reactor and the second liquid vapor separator are operated at temperature and pressure within 10% of each other, where pressure is on an absolute basis and temperature is on a Celsius scale.

9. The hydroformylation system for making aldehydes according to Claim 1, wherein the olefin feed comprises propylene and the aldehyde product comprises butyraldehyde.

10. The hydroformylation system for making aldehydes according to Claim 1, wherein the catalyst is a Group **VIII** metal complexed with a phosphorous containing ligand and carbon monoxide.

11. The hydroformylation system for making aldehydes according to Claim 10, wherein the Group **VIII** metal is rhodium.

12. A method of making aldehydes from olefins and syngas by way of hydroformylation comprising:
(a) feeding catalyst, syngas and olefin to a primary reactor;
(b) converting the syngas and olefin to a product aldehyde in the primary reactor;
(c) providing the product aldehyde and unreacted syngas and unreacted olefin from the primary reactor to a first liquid vapor separator;
(d) separating the product aldehyde and unreacted olefin into a liquid crude aldehyde product stream and a vaporous vent stream containing unreacted syngas and olefin;
(e) feeding the vaporous vent stream from the first liquid vapor separator and catalyst from the primary reactor to a vent reactor;
(f) converting unreacted olefin and syngas from the vaporous vent stream received from the first liquid vapor separator to additional product aldehyde in the vent reactor;
(g) feeding the entire output from the vent reactor to a second liquid vapor separator;
(h) separating output from the vent reactor into a liquid stream containing additional product aldehyde and catalyst and an offgas stream;
(i) returning the liquid stream containing additional product aldehyde and catalyst from the second liquid vapor separator to the primary reactor,
whereby catalyst from the second liquid vapor separator is recycled to the primary reactor and additional product aldehyde from the second liquid vapor separator is provided to the primary reactor and is recovered in the crude aldehyde product stream from the first liquid vapor separator.

13. The method of making aldehydes from olefins and syngas by way of hydroformylation according to Claim 12, further comprising cooling the output from the vent reactor.

14. The method of making aldehydes from olefins and syngas by way of hydroformylation according to Claim 12, wherein the output from the vent reactor is separated into a liquid stream containing additional product aldehyde and catalyst and an offgas stream without cooling.

15. The method of making aldehydes from olefins and syngas by way of hydroformylation according to Claim 12, wherein hydroformylation is carried out in the primary and vent reactors while maintaining a temperature of from 120°C to 140°C in the reactors.

## Patentansprüche

1. Hydroformylierungssystem zur Herstellung von Aldehyden, umfassend:
(a) einen Primärreaktor, der mit Katalysatorzufuhr, Synthesegaszufuhr und Olefinzufuhr versehen ist und dazu eingerichtet ist, das Olefin und Synthesegas in Produktaldehyd umzuwandeln;
(b) einen ersten Flüssigkeits-Dampf-Abscheider, der mit dem Primärreaktor gekoppelt ist zum Aufnehmen von Austrag aus diesem und dazu eingerichtet ist, den Produktaldehyd in einen Rohaldehydproduktstrom und einen Entlüftungsstrom, der Synthesegas und nicht umgesetztes Olefin enthält, zu trennen;
(c) einen Entlüftungsreaktor, der mit dem ersten Flüssigkeits-Dampf-Abscheider gekoppelt ist, um den Entlüftungsstrom aus diesem aufzunehmen, wobei der Entlüftungsreaktor außerdem mit dem Primärreaktor gekoppelt ist, der dazu konfiguriert ist, Katalysator an diesen bereitzustellen, wobei der Entlüftungsreaktor dazu eingerichtet ist, nicht umgesetztes Olefin in dem Entlüftungsstrom aus dem ersten Flüssigkeits-Dampf-Abscheider in zusätzlichen Produktaldehyd umzuwandeln;
(d) einen zweiten Flüssigkeits-Dampf-Abscheider, der mit dem Entlüftungsreaktor so gekoppelt ist, dass er den gesamten Austrag aus dem Entlüftungsreaktor aufnimmt, und dazu eingerichtet ist, den Austrag aus dem Entlüftungsreaktor in einen flüssigen Rückführstrom, der zusätzlichen Produktaldehyd und Katalysator enthält, sowie einen weiteren Entlüftungsstrom zu trennen, wobei der zweite Flüssigkeits-Dampf-Abscheider außerdem mit dem Primärreaktor so gekoppelt ist, dass er diesem den Rückführstrom bereitstellt;
wodurch Katalysator aus dem zweiten Flüssigkeits-Dampf-Abscheider in den Primärreaktor rückgeführt wird und zusätzlicher Produktaldehyd aus dem zweiten Flüssigkeits-Dampf-Abscheider dem Primärreaktor bereitgestellt wird und in dem Rohaldehydproduktstrom aus dem ersten Flüssigkeits-Dampf-Abscheider gewonnen wird.

2. Hydroformylierungssystem zur Herstellung von Aldehyden nach Anspruch 1, wobei das Hydroformylierungssystem aus Folgendem besteht: (a) dem Primärreaktor; (b) dem ersten Flüssigkeits-Dampf-Abscheider; (c) dem Entlüftungsreaktor; und (d) dem zweiten Flüssigkeits-Dampf-Abscheider.

3. Hydroformylierungssystem zur Herstellung von Aldehyden nach Anspruch 1, wobei das System einen einzigen Rohaldehydproduktauslass aufweist.

4. Hydroformylierungssystem zur Herstellung von Aldehyden nach Anspruch 1, wobei der Entlüftungsstrom aus dem ersten Flüssigkeits-Dampf-Abscheider Produktaldehyd, Synthesegas und nicht umgesetztes Olefin umfasst.

5. Hydroformylierungssystem zur Herstellung von Aldehyden nach Anspruch 1, wobei der Entlüftungsreaktor ein Pfropfenströmungsreaktor ist.

6. Hydroformylierungssystem zur Herstellung von Aldehyden nach Anspruch 1, wobei der Entlüftungsreaktor ein rückvermischter Reaktor ist.

7. Hydroformylierungssystem zur Herstellung von Aldehyden nach Anspruch 1, wobei der zweite Dampf-Flüssigkeits-Abscheider mit dem Entlüftungsreaktor in einem gemeinsamen Mantel integriert ist.

8. Hydroformylierungssystem zur Herstellung von Aldehyden nach Anspruch 7, wobei der Primärreaktor, der Entlüftungsreaktor und der zweite Flüssigkeits-Dampf-Abscheider bei Temperatur und Druck innerhalb von 10 % zueinander betrieben werden, wobei Druck auf absoluter Basis und Temperatur auf der Celsius-Skala bezogen sind.

9. Hydroformylierungssystem zur Herstellung von Aldehyden nach Anspruch 1, wobei die Olefinzufuhr Propylen umfasst und das Aldehydprodukt Butyraldehyd umfasst.

10. Hydroformylierungssystem zur Herstellung von Aldehyden nach Anspruch 1, wobei der Katalysator ein Metall der Gruppe VIII ist, das mit einem phosphorhaltigen Liganden und Kohlenmonoxid komplexiert ist.

11. Hydroformylierungssystem zur Herstellung von Aldehyden nach Anspruch 10, wobei das Metall der Gruppe VIII Rhodium ist.

12. Verfahren zur Herstellung von Aldehyden aus Olefinen und Synthesegas mittels Hydroformylierung, umfassend:
(a) Zuführen von Katalysator, Synthesegas und Olefin zu einem Primärreaktor;
(b) Umwandeln des Synthesegases und Olefins in einen Produktaldehyd in dem Primärreaktor;
(c) Bereitstellen des Produktaldehyds und von nicht umgesetztem Synthesegas und nicht umgesetztem Olefin aus dem Primärreaktor an einen ersten Flüssigkeits-Dampf-Abscheider;
(d) Trennen des Produktaldehyds und von nicht umgesetztem Olefin in einen flüssigen Rohaldehydproduktstrom und einen dampfförmigen Entlüftungsstrom, der nicht umgesetztes Synthesegas und Olefin enthält;
(e) Zuführen des dampfförmigen Entlüftungsstroms aus dem ersten Flüssigkeits-Dampf-Abscheider und von Katalysator aus dem Primärreaktor zu einem Entlüftungsreaktor;
(f) Umwandeln von nicht umgesetztem Olefin und Synthesegas aus dem von dem ersten Flüssigkeits-Dampf-Abscheider empfangenen dampfförmigen Entlüftungsstrom in zusätzlichen Produktaldehyd in dem Entlüftungsreaktor;
(g) Zuführen des gesamten Austrags aus dem Entlüftungsreaktor zu einem zweiten Flüssigkeits-Dampf-Abscheider;
(h) Trennen von Austrag aus dem Entlüftungsreaktor in einen flüssigen Strom, der zusätzlichen Produktaldehyd und Katalysator enthält, und einen Abgasstrom;
(i) Zurückleiten des flüssigen Stroms, der zusätzlichen Produktaldehyd und Katalysator enthält, aus dem zweiten Flüssigkeits-Dampf-Abscheider zu dem Primärreaktor,
wodurch Katalysator aus dem zweiten Flüssigkeits-Dampf-Abscheider in den Primärreaktor rückgeführt wird und zusätzlicher Produktaldehyd aus dem zweiten Flüssigkeits-Dampf-Abscheider dem Primärreaktor bereitgestellt wird und in dem Rohaldehydproduktstrom aus dem ersten Flüssigkeits-Dampf-Abscheider gewonnen wird.

13. Verfahren zur Herstellung von Aldehyden aus Olefinen und Synthesegas mittels Hydroformylierung nach Anspruch 12, ferner umfassend Kühlen des Austrags aus dem Entlüftungsreaktor.

14. Verfahren zur Herstellung von Aldehyden aus Olefinen und Synthesegas mittels Hydroformylierung nach Anspruch 12, wobei der Austrag aus dem Entlüftungsreaktor in einen flüssigen Strom, der zusätzlichen Produktaldehyd und Katalysator enthält, und einen Abgasstrom ohne Kühlung getrennt wird.

15. Verfahren zur Herstellung von Aldehyden aus Olefinen und Synthesegas mittels Hydroformylierung nach Anspruch 12, wobei Hydroformylierung in dem Primär- und dem Entlüftungsreaktor unter Aufrechterhaltung einer Temperatur von 120 °C bis 140 °C in den Reaktoren durchgeführt wird.

## Revendications

1. Système d'hydroformylation pour la fabrication d'aldéhydes comprenant:
(a) un réacteur primaire doté d'une alimentation en catalyseur, d'une alimentation en gaz de synthèse et d'une alimentation en oléfine, conçu pour transformer l'oléfine et le gaz de synthèse en produit d'aldéhyde;
(b) un premier séparateur liquide-vapeur couplé au réacteur primaire pour recevoir l'effluent de celui-ci, conçu pour séparer le produit d'aldéhyde en un flux de produit d'aldéhyde brut et un flux d'évent contenant du gaz de synthèse et de l'oléfine n'ayant pas réagi;
(c) un réacteur à évent couplé au premier séparateur liquide-vapeur pour recevoir le flux d'évent de celui-ci, le réacteur à évent étant également couplé au réacteur primaire qui est configuré pour fournir un catalyseur à celui-ci, dans lequel le réacteur à évent est conçu pour convertir l'oléfine n'ayant pas réagi dans le flux d'évent provenant du premier séparateur liquide-vapeur en un produit d'aldéhyde supplémentaire;
(d) un deuxième séparateur liquide-vapeur couplé au réacteur à évent de manière à recevoir la totalité de l'effluent du réacteur à évent et conçu pour séparer l'effluent du réacteur à évent en un flux de recyclage liquide contenant un produit d'aldéhyde supplémentaire et un catalyseur ainsi qu'un autre flux d'évent, le deuxième séparateur liquide-vapeur étant également couplé au réacteur primaire de manière à fournir le flux de recyclage à celui-ci;
dans lequel le catalyseur provenant du deuxième séparateur liquide-vapeur est recyclé vers le réacteur primaire et le produit d'aldéhyde supplémentaire provenant du deuxième séparateur liquide-vapeur est acheminé vers le réacteur primaire et récupéré dans le flux de produit d'aldéhyde brut provenant du premier séparateur liquide-vapeur.

2. Système d'hydroformylation pour la fabrication d'aldéhydes selon la revendication 1, dans lequel le système d'hydroformylation est constitué de: (a) le réacteur primaire; (b) le premier séparateur liquide-vapeur; (c) le réacteur à évent; d) le deuxième séparateur liquide-vapeur.

3. Système d'hydroformylation pour la fabrication d'aldéhydes selon la revendication 1, dans lequel le système comporte une unique sortie pour le produit d'aldéhyde brut.

4. Système d'hydroformylation pour la fabrication d'aldéhydes selon la revendication 1, dans lequel le flux d'évent provenant du premier séparateur liquide-vapeur comprend un produit d'aldéhyde, du gaz de synthèse, et de l'oléfine n'ayant pas réagi.

5. Système d'hydroformylation pour la fabrication d'aldéhydes selon la revendication 1, dans lequel le réacteur à évent est un réacteur à écoulement piston.

6. Système d'hydroformylation pour la fabrication d'aldéhydes selon la revendication 1, dans lequel le réacteur à évent est un réacteur à mélange complet.

7. Système d'hydroformylation pour la fabrication d'aldéhydes selon la revendication 1, dans lequel le deuxième séparateur liquide-vapeur est intégré au réacteur à évent à l'intérieur d'une enveloppe commune.

8. Système d'hydroformylation pour la fabrication d'aldéhydes selon la revendication 7, dans lequel le réacteur primaire, le réacteur à évent et le deuxième séparateur liquide-vapeur fonctionnent à une température et à une pression dont les écarts ne dépassent pas 10 % les uns par rapport aux autres, la pression étant exprimée en pression absolue et la température selon l'échelle Celsius.

9. Système d'hydroformylation pour la fabrication d'aldéhydes selon la revendication 1, dans lequel l'alimentation en oléfine comprend du propylène et le produit d'aldéhyde comprend du butyraldéhyde.

10. Système d'hydroformylation pour la fabrication d'aldéhydes selon la revendication 1, dans lequel le catalyseur est un métal du Groupe VIII complexé avec du monoxyde de carbone et avec un ligand contenant du phosphore.

11. Système d'hydroformylation pour la fabrication d'aldéhydes selon la revendication 10, dans lequel le métal du Groupe VIII est le rhodium.

12. Procédé de fabrication d'aldéhydes à partir d'oléfines et de gaz de synthèse par hydroformylation comprenant:
a) l'alimentation d'un réacteur primaire en catalyseur, en gaz de synthèse et en oléfine;
b) la conversion du gaz de synthèse et de l'oléfine en un produit d'aldéhyde dans le réacteur primaire;
c) l'acheminement du produit d'aldéhyde, du gaz de synthèse n'ayant pas réagi et l'oléfine n'ayant pas réagi depuis le réacteur primaire vers un premier séparateur liquide-vapeur;
d) la séparation du produit d'aldéhyde et de l'oléfine n'ayant pas réagi en un flux de produit d'aldéhyde brut liquide et un flux d'évent vaporeux contenant du gaz de synthèse et de l'oléfine n'ayant pas réagi;
e) l'acheminement du flux d'évent vaporeux provenant du premier séparateur liquide-vapeur et du catalyseur depuis le réacteur primaire vers un réacteur à évent;
f) la conversion de l'oléfine n'ayant pas réagi et du gaz de synthèse provenant du flux d'évent vaporeux reçu du premier séparateur liquide-vapeur en un produit d'aldéhyde supplémentaire dans le réacteur à évent;
g) l'acheminement de la totalité de l'effluent du réacteur à évent vers un deuxième séparateur liquide-vapeur;
h) la séparation de l'effluent du réacteur à évent en un flux liquide contenant un produit d'aldéhyde supplémentaire et un catalyseur et un flux de gaz résiduaire;
(i) le renvoi du flux liquide contenant le produit d'aldéhyde supplémentaire et le catalyseur depuis le deuxième séparateur liquide-vapeur vers le réacteur primaire,
dans lequel le catalyseur provenant du deuxième séparateur liquide-vapeur est recyclé vers le réacteur primaire et le produit d'aldéhyde supplémentaire provenant du deuxième séparateur liquide-vapeur est acheminé vers le réacteur primaire et récupéré dans le flux produit d'aldéhyde brut provenant du premier séparateur liquide-vapeur.

13. Procédé de fabrication d'aldéhydes à partir d'oléfines et de gaz de synthèse par hydroformylation selon la revendication 12, comprenant en outre le refroidissement de l'effluent du réacteur à évent.

14. Procédé de fabrication d'aldéhydes à partir d'oléfines et de gaz de synthèse par hydroformylation selon la revendication 12, dans lequel l'effluent du réacteur à évent est séparé en un flux liquide contenant un produit d'aldéhyde supplémentaire et un catalyseur et un flux de gaz résiduaire sans refroidissement.

15. Procédé de fabrication d'aldéhydes à partir d'oléfines et de gaz de synthèse par hydroformylation selon la revendication 12, dans lequel l'hydroformylation est effectuée dans les réacteurs primaire et à évent tout en maintenant une température comprise entre 120°C et 140°C dans les réacteurs.
